# FASCICULE DE BREVET EUROPEEN

(11) **EP 3 547 911 B1**
(45) Date de publication et mention de la délivrance du brevet: **15.02.2023**
(21) Numéro de dépôt: 17816987.6
(22) Date de dépôt: 05.12.2017
(51) Int. Cl.: A61B 5/372

(54) **PROCEDES ET DISPOSITIFS DE DETERMINATION D'UN SIGNAL SYNTHETIQUE D'UNE ACTIVITE BIOELECTRIQUE**
VERFAHREN UND VORRICHTUNGEN ZUR BESTIMMUNG EINES SYNTHETISCHEN SIGNALS EINER BIOELEKTRISCHEN AKTIVITÄT
METHODS AND DEVICES FOR DETERMINING A SYNTHETIC SIGNAL OF A BIOELECTRIC ACTIVITY

(30) Priorité: 05.12.2016 FR 1661958
(43) Date de publication de la demande: 09.10.2019
(73) Titulaire: DREEM, 75009 Paris (FR)
(72) Inventeur: SOULET DE BRUGIÈRE, Quentin, 33115 Pyla sur Mer (FR); MERCIER, Hugo, 75008 Paris (FR); THOREY, Valentin, 75015 Paris (FR); GALTIER, Mathieu, 44000 Nantes (FR); KALIFA, Jérôme, 92160 Antony (FR)
(74) Mandataire: Plasseraud IP
(86) Numéro de dépôt international: PCT/FR2017/053383
(87) Numéro de publication internationale: WO 2018/104645

(56) Documents cités:
- WO-A1-2011/007292
- WO-A2-2007/052108
- FR-A1- 2 923 150
- US-A1- 2012 071 744
- US-A1- 2014 051 044
- US-A1- 2016 302 684

## Description

La présente invention est relative aux procédés et aux dispositifs de détermination d'un signal synthétique robuste indicatif d'une activité bioélectrique d'un individu, notamment indicatif d'une activité électroencéphalographique.

De tels procédé et systèmes peuvent notamment être utilisés pour déterminer un signal électroencéphalographique (EEG) robuste à partir d'un dispositif d'acquisition EEG comportant par exemple des électrodes sèches.

Les documents suivants traitent de sujets connexes : US 2014/051044, US 2016/302684, US 2012/071744 et FR 2 923 150, WO 2007/052108 A2.

L'invention trouve une application toute particulière dans le domaine de l'acquisition et du traitement temps réel des signaux bioélectriques, notamment par des dispositifs portables et autonomes d'acquisition et de traitement des signaux.

Un enjeu majeur de l'acquisition dans le cadre d'un dispositif portable de mesure de signaux bioélectriques, en particulier à partir d'électrode sèche, est la robustesse de l'acquisition. Ainsi, il arrive régulièrement qu'une ou plusieurs voies d'acquisitions se déconnectent ou ne fournisse pas un signal de qualité optimale. La qualité de l'acquisition peut alors fortement varier en fonction de l'état du contact entre l'électrode et la peau notamment.

La présente invention a notamment pour but de pallier ces inconvénients.

La présente invention décrit des méthodes pour obtenir un signal robuste correspondant à une activité bioélectrique, par exemple l'activité cérébrale par recombinaison, de signaux provenant d'électrodes localisée à proximité les unes des autres.

L'invention repose sur le fait que des électrodes spatialement proches captent des signaux bioélectriques similaires. En particulier les rythmes les motifs caractéristiques observés dans les signaux bioélectriques surviennent en même temps dans une zone spatiale réduite.

A cet effet, l'invention a pour premier objet un procédé de détermination d'un signal synthétique robuste selon la revendication 1.

Dans des modes de réalisation préférés de l'invention, on peut éventuellement avoir recours en outre à l'une et/ou à l'autre des dispositions suivantes :
- chaque signal de mesure est obtenu à partir d'une mesure différentielle entre deux électrodes de mesure, entre une électrode de mesure et une électrode de biais, ou encore entre une électrode de biais et une masse interne d'une unité de traitement des signaux de mesure;
- les séries temporelles d'indices de confiance sont construites en fonction d'au moins l'un parmi la puissance du signal de mesure, la puissance de la dérivée du signal de mesure, des puissances de bandes de fréquences du signal de mesure, un taux de passages par zéro du signal de mesure, une détection de saturations du signal de mesure;

- pour déterminer le signal synthétique, on détermine une série temporelle de valeurs de signal synthétique telle que à chaque instant de la série temporelle, la valeur de signal synthétique correspond à la valeur du signal de mesure associé à l'indice de confiance le plus élevé pour ledit instant;
- pour déterminer le signal synthétique, on détermine une série temporelle de valeurs de signal synthétique telle que à chaque instant de la série temporelle, la valeur de signal synthétique correspond à une moyenne pondérée des valeurs des signaux de mesure, chaque valeur des signaux de mesure étant respectivement pondérée en fonction de l'indice de confiance associé au signal de mesure pour ledit instant;
- le signal synthétique comporte une liste de motifs prédéfinis d'activité bioélectrique associés à des instants temporels ou des fenêtres temporelles d'acquisition;
- pour déterminer le signal synthétique, on construit la liste de motifs constituant le signal synthétique à partir des listes de motifs associées à chaque signal de mesure, chaque motif de chacune desdites listes de motifs étant pondéré par l'indice de confiance associé au signal de mesure associé pour l'instant temporel ou la fenêtre temporelle d'enregistrement associé(e) audit motif; - on détermine une référence virtuelle à partir d'une mesure de potentiel d'une électrode de mesure ou d'une électrode de biais et en fonction d'une mesure d'un courant échangé entre une électrode de biais et l'individu.

L'invention a également pour objet un dispositif d'acquisition d'un signal synthétique robuste selon la revendication 9.

D'autres caractéristiques et avantages de l'invention apparaîtront au cours de la description suivante d'une de ses formes de réalisation, donnée à titre d'exemple non limitatif, en regard des dessins joints.

Sur les dessins :
- la figure 1 et la figure 2 sont des vues schématique d'un dispositif d'acquisition d'un signal synthétique robuste indicatif d'une activité bioélectrique d'un individu,
- la figure 3 est un organigramme illustrant un premier mode de réalisation d'un procédé de détermination d'un signal synthétique robuste indicatif d'une activité bioélectrique d'un individu,
- la figure 4 est un organigramme illustrant un deuxième mode de réalisation d'un procédé de détermination d'un signal synthétique robuste indicatif d'une activité bioélectrique d'un individu.

Sur les différentes figures, les mêmes références désignent des éléments identiques ou similaires.

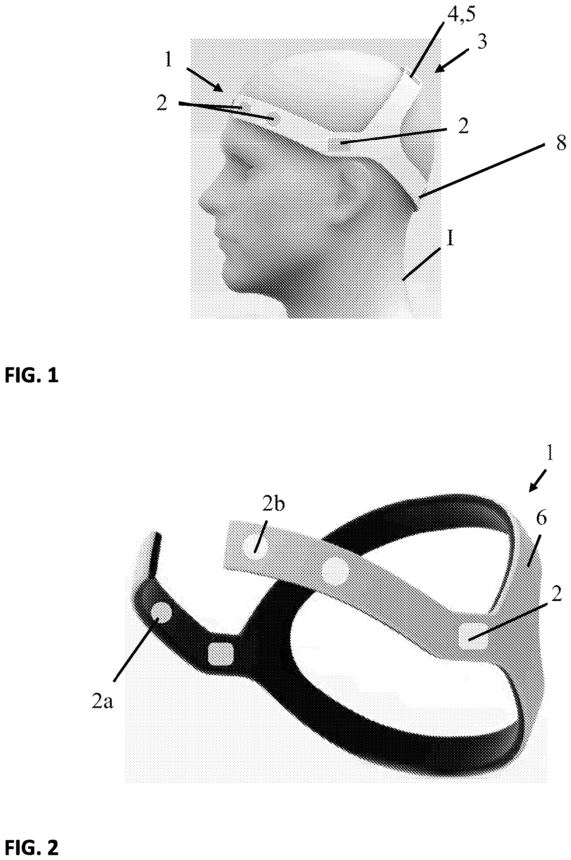

La figure 1 illustre un dispositif 1 d'acquisition d'un signal synthétique robuste indicatif d'une activité bioélectrique d'un individu I

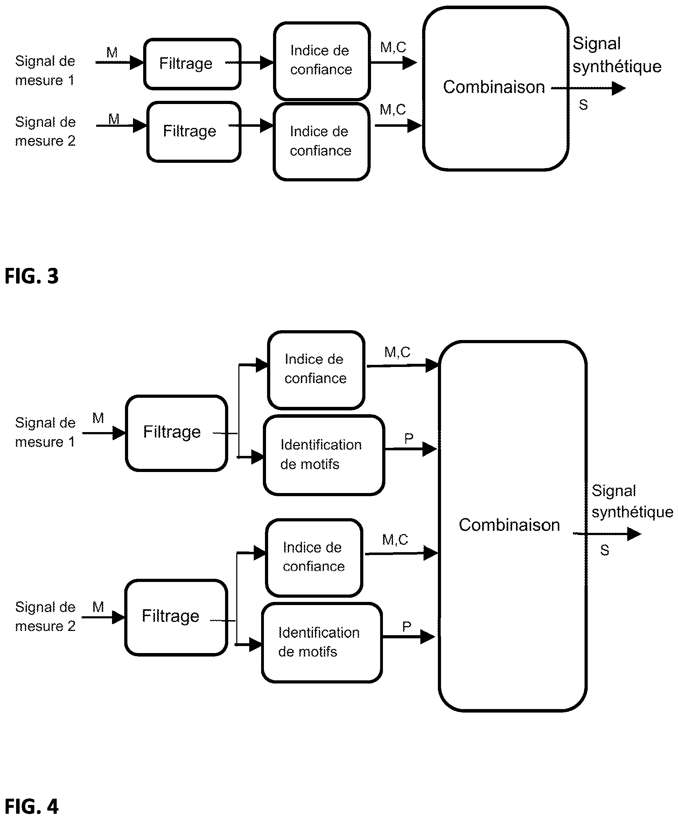

Un tel dispositif 1 est apte à mettre en oeuvre un procédé de détermination d'un signal synthétique robuste indicatif d'une activité bioélectrique d'un individu , par exemple tel qu'illustré sur la figure 3.

Comme cela est visible sur la figure 1, le dispositif 1 comprend au moins deux électrodes 2. Les électrodes sont placées en contact, ou en proximité, avec l'individu I.

Les électrodes 2 comprennent des électrodes de mesure 2b qui permettent d'acquérir continument au moins deux signaux de mesure M respectivement représentatifs de deux signaux électriques physiologiques dans l'individu I.

Les électrodes peuvent comprendre une électrode de biais 2b adaptée pour maintenir un potentiel prédéfini de la tête par rapport à une masse du dispositif 1.

Les signaux de mesure sont déterminés à partir de mesures des potentiels des électrodes 2 et en particulier déterminés par une mesure d'une différence de potentiels entre deux électrodes.

Par « potentiel », on entend donc un signal issu d'une électrode unique et par « dérivation » (par exemple celles détaillées ci-dessous, on entend la différence de deux potentiels.

Un signal de mesure est par exemple :
- une dérivation entre des électrodes de mesure relativement éloignées, par exemple Mastoïde / Front,
- une dérivation entre des électrodes de mesure adjacentes ou proches (Front-Front, Cheveux-cheveux, Front-Cheveux),
- une dérivation entre une électrode (par exemple Front) et une électrode de biais. Le signal d'une électrode de biais est similaire à celui d'une électrode de mesure, on peut donc en exploiter les caractéristiques. Par ailleurs, dans le cas du décollement d'une électrode de mesure, par exemple front gauche (Fp1) (respectivement front droit (Fp2)), si la dérivation Fp2-biais (respectivement Fp1-biais) est mesurée alors on peut utiliser ce dernier signal pour caractériser les signaux électriques physiologiques.
- une dérivation entre deux électrodes de mesure et/ou de biais, modifiée par une opération analogique (par exemple filtrage ou modulation), ou modulé par la mesure du courant en sortie du biais vers le circuit électronique, pour obtenir une référence virtuelle,
- un signal issu de la combinaison de plusieurs dérivations, telles qu'indiquées ci-avant, par exemple au moyen d'une opération analogique (telle qu'un filtrage, une modulation, une moyenne, etc.) permettant par exemple d'en faire une référence virtuelle, et/ou de la combinaison avec un signal tiers utilisé pour analyser une activité physiologique (par exemple le sommeil) et des événements caractéristiques de ladite activité physiologique.

Ainsi par exemple, il est possible de moduler le potentiel provenant d'une électrode de mesure ou du biais en fonction du courant en sortie du biais vers le circuit électronique. Ceci permet par exemple de corriger le potentiel provenant d'une électrode pour qu'il corresponde à une référence virtuelle.

Par « référence virtuelle », on entend un potentiel qui est similaire à un signal mesuré en un point du corps humain éloigné ou protégé des ondes cérébrales, par exemple un signal similaire à un signal provenant d'une électrode placée sur un mastoïde ou un lobe d'oreille.

Une référence virtuelle peut être déterminée à partir d'un potentiel mesuré en un point, du corps humain ou du circuit électronique du dispositif, qui est différent d'une référence classique pour la mesure de signaux bioélectrique (par exemple d'électroencéphalogramme) comme la mastoïde, le lobe d'oreille ou le nez. Le potentiel brut mesuré à un tel point est ainsi corrigé pour se rapprocher du potentiel attendu pour une référence, c'est-à-dire de sorte à être indépendant, dans la mesure du possible, des ondes cérébrales. La mesure à un tel point est avantageusement plus facile d'accès ou plus confortable (par exemple le front) que la mesure à un point de référence classiquement utilisé comme le lobe d'oreille.

Il est alors possible d'obtenir un signal de mesure à partir d'une électrode de mesure et de la référence virtuelle.

Les signaux de mesures peuvent être issus de dérivations ayant des électrodes en commun ou pas.

Par exemple trois signaux de mesures peuvent être acquis correspondant respectivement aux dérivations suivantes :
- front gauche - front droit
- tempe gauche - front gauche
- tempe droite - front droit.

Le dispositif 1 comporte par ailleurs une unité de traitement 3 comprenant des moyens de réception 4 des signaux de mesure des électrodes 2, et des moyens de traitement 5.

Les moyens de traitement 5 peuvent par exemple comporter une ou plusieurs processeurs ainsi que une ou plusieurs mémoires appropriées.

Les moyens de réception 4 peuvent par exemple comporter des connecteurs ainsi qu'une électronique de communication et/ou de contrôle des électrodes.

Les moyens de traitement 5 et les moyens de réception 4 peuvent être intégrés dans une puce unique.

Le dispositif peut comporter un châssis 6 unique sur lequel sont monté les électrodes 2, l'unité de traitement 3, les moyens de réception 4 et les moyens de traitement 5. Le dispositif 1 peut encore comporter une batterie. La batterie 8 peut être montée sur le châssis 6.

Les moyens de traitement 5 sont adaptés pour
- construire continument, à partir des signaux de mesure M, au moins deux séries temporelles d'indices de confiance C respectivement associées auxdits signaux de mesure M,
- déterminer continument un signal synthétique S à partir desdits signaux de mesure et desdites séries temporelles d'indices de confiance comme cela va maintenant être décrit plus en détail.

On se réfère à présent plus particulièrement aux figures 3 et 4 qui décrivent différents procédés de détermination d'un signal synthétique robuste indicatif d'une activité bioélectrique d'un individu.

Dans un premier mode de réalisation , illustré sur la figure 3, le signal synthétique comporte des valeurs des signaux de mesure, le cas échéant pondérées, comme cela est détaillé ci-après.

Dans un second mode de réalisation , illustré sur la figure 4, le signal synthétique comporte une liste de motifs prédéfinis d'activité bioélectrique associés à des instants temporels ou des fenêtres temporelles d'acquisition.

De tels motifs prédéfinis d'activité bioélectrique sont par exemple des oscillations lentes (« slow oscillations »), des ondes sigma (« spindles »), des K-complexes, des rythmes lents, des micro réveils, des rythmes alpha, etc...

On se réfère tout d'abord à la figure 3 qui illustre le premier mode de réalisation , donné à titre indicatif . Dans ce mode de réalisation, le signal synthétique comporte des valeurs, le cas échant pondérées, de plusieurs signaux électriques physiologiques proches pour obtenir un signal de bonne qualité.

Par « signaux électriques physiologiques proches », on entend ainsi par exemple des signaux de mesure provenant d'électrodes placées à des positions proches, par exemple de deux électrodes placées en position frontale. Les électrodes proches sont par exemple espacées de moins de 10 centimètres, par exemple de moins de 5 centimètres.

Par « signal de bonne qualité », on entend la détection et mise à l'écart des signaux de mauvaise qualité et/ou la combinaison éventuelle des signaux similaires de bonne qualité. La qualité du signal peut notamment être quantifiée comme cela va être décrit ci-après.

Une première étape du procédé peut comporter des étapes de filtrages. Par exemple un filtrage du 50Hz ou du 60Hz, des fréquences supérieures à 200Hz, et éventuellement d'autres bandes. Différentes méthodes de filtrages sont possibles, par exemple un filtrage Butterworth, à ondelette ou de Kalman.

La méthode de quantification de la qualité du signal est adaptée pour permettre de reconnaître des amplitudes du signal ou des formes d'ondes anormales (c'est à dire ne correspondant par exemple pas à un signal encéphalographique usuellement observé).

Pour cela, on construit continument, pour chaque signal de mesure, une série temporelle d'indices de confiance associée audit signal de mesure.

Par « construit continument », on entend que les séries d'indicateur de confiance sont construites en temps réel (par exemple en temps réel souple) simultanément et en parallèle avec l'acquisition desdits signaux de mesure.

Par « continûment », on entend que les opérations et/ou étapes sont répétées dans le temps au cours d'une période d'acquisition des signaux de mesure par le dispositif, notamment répétées d'une manière périodique ou quasi-périodiques.

Chaque indice de confiance peut être calculé à partir d'une grandeur dérivée du signal de mesure, par exemple choisie parmi la puissance du signal de mesure, la puissance de la dérivée du signal de mesure, les différentes bandes de fréquences du signal de mesure, la valeur de l'impédance du signal de mesure, le taux de passages par zéro du signal de mesure, la détection de saturations d'un amplificateur de la chaîne d'acquisition du signal de mesure, les valeurs du signal de mesure.

Dans un mode de réalisation, on peut acquérir en outre au moins un signal de mesure additionnel représentatif d'une activité physiologique de l'individu. Le signal de mesure additionnel peut par exemple être mesuré par un accéléromètre.

L'indice de confiance d'un signal de mesure peut également être indicatif d'une confiance dans les données d'autres signaux de mesure. Ainsi par exemple le signal de mesure obtenu par un accéléromètre, témoignant des mouvements de l'utilisateur, peut être utilisé pour déterminer un indice de confiance dans les valeurs mesurées par des électrodes.

Dans un exemple de réalisation donné à titre indicatif, le dispositif peut exploiter la présence de la fréquence du secteur de distribution électrique, 50 Hz, pour détecter le décollement d'une électrode et éliminer un signal. Ainsi, lorsque l'on mesure une dérivation entre deux électrodes en contact, ou en proximité pour des électrodes de proximité, avec l'utilisateur, par exemple FP1-FP2, les fréquences 50Hz présentes sur les deux signaux de mesure se compensent en grande partie. Lorsqu'une des électrodes se décolle, ou s'éloigne dans le cas d'électrodes de proximité, la puissance du signal dans la bande 50 Hz augmente significativement pour ladite électrode. Le signal dérivé de FP1-FP2 comprend alors une puissance du 50 Hz très élevé. Ceci permet de détermine un indice de confiance du signal.

Les indices et/ou les grandeurs dérivées du signal de mesure peuvent être calculés sur des fenêtres temporelles de durée variable, selon l'indicateur visé.

Les indices peuvent comporter des valeurs binaires (par exemple, un booléen qui dit si oui ou non le signal est de bonne qualité), catégorielles ou des valeurs variant continûment sur une plage prédéfinie (par exemple quantifiant la probabilité d'avoir un signal de bonne qualité).

L'opération de construction d'un indice de confiance à partir d'un ou de plusieurs signaux de mesure peut comporter la détermination d'une ou plusieurs grandeurs indiquées ci-dessus puis leur comparaison avec un ou plusieurs seuil(s) prédéfinis, par exemple à partir de valeurs usuellement rencontrées en électro-encéphalographie.

L'opération de construction d'un indice de confiance peut également employer un prédicteur obtenu par une méthode d'apprentissage (par exemple une forêt aléatoire ou un réseau de neurones) et entraîné sur une base d'apprentissage construite à partir d'extraits de signaux de mesure labellisés comme de bonne ou de mauvaise qualité. Cette base d'apprentissage peut inclure des valeurs indices (par exemple ceux présentés précédemment) calculés sur les mêmes signaux labellisés.

Une fois un indice de confiance déterminé, le procédé comporte la détermination continue un signal synthétique à partir des signaux de mesure et des séries temporelles d'indices de confiance.

Ceci permet d'écarter en temps réel les signaux de mauvaise qualité. Plusieurs variantes de réalisation sont envisageables pour construire le signal synthétique à partir des signaux à un instant donné.

Selon une première variante, le signal synthétique à un instant donné est obtenu à partir du signal de mesure présentant la meilleure qualité audit instant. Si la qualité diminue en dessous d'un seuil à un instant ultérieur, le signal synthétique est déterminé à partir du signal de mesure présentant la meilleure qualité audit instant ultérieur.

Selon une deuxième variante, le signal synthétique à un instant donné est obtenus par une combinaison des signaux de mesure audit instant présentant une qualité suffisante indiquée par l'indice de confiance. De cette manière, il est possible de moyenner les signaux de mesure acquis à des positions proches sur l'individu pour augmenter la robustesse du signal synthétique résultant.

Le signal synthétique est alors déterminé par une moyenne des signaux de mesure acquis à des positions proches et présentant une qualité suffisante indiquée par l'indice de confiance. La moyenne peut être pondérée par l'indice de confiance de chaque signal de mesure à l'instant associé.

D'autres stratégies de combinaison des signaux de mesure sont bien évidemment envisageables.

Par ailleurs, comme indiqué ci-dessus, l'indice de confiance associé à un instant donné à un signal de mesure peut être déterminé à partir d'un autre signal de mesure, par exemple un signal de mesure additionnel représentatif d'une activité physiologique de l'individu comme celui fourni par un accéléromètre.

On se réfère à présent à la figure 4 qui illustre le deuxième mode de réalisation . Selon l'invention et dans ce mode de réalisation, le signal synthétique comporte une liste de motifs prédéfinis d'activité bioélectrique associés à des instants temporels ou des fenêtres temporelles d'acquisition.

Ce deuxième mode de réalisation peut être opéré en combinaison avec le premier mode de réalisation, par exemple en étant mis en oeuvre en parallèle du premier mode de réalisation décrit ci-dessus.

Ce deuxième mode de réalisation permet d'obtenir directement des évènements d'intérêt identifiés dans les signaux de mesure en réalisant les détections et analyses sur chaque signal de mesure avant de combiner les détections en fonction des indices de confiance.

Dans ce mode de réalisation, on calcule à nouveau un indice de confiance comme indiqué ci-dessus.

Puis, ou parallèlement, on traite le signal de mesure pour identifier des motifs prédéfinis d'activité bioélectrique. On associe alors à chacun des signaux de mesure une liste de motifs identifiés, chaque motif identifié étant associé à un instant temporel ou une fenêtre temporelle d'enregistrement dans le signal de mesure associé.

La liste des motifs identifiés comprend par exemple des motifs associés à des oscillations lentes (« slow oscillations »), des ondes sigma (« spindles »), des K-complexes, des rythmes lents, des micro réveils, des rythmes alpha, etc.

Pour chaque voie d'acquisition, i.e. chaque signal de mesure, ayant une qualité correcte indiquée par l'indice de confiance, on définit alors une probabilité que chacun de ces évènements ait lieu. Ces probabilités sont ensuite comparées pour déterminer la présence de l'un des évènements associé aux motifs.

En particulier, pour chaque instant temporel ou fenêtre temporelle d'enregistrement d'un signal de mesure, et pour chaque motif de la liste de motifs identifiés, on détermine une probabilité que l'évènement associé au motif ait lieu, c'est-à-dire que l'activité bioélectrique associée au motif se soit produite pendant l'instant temporel ou pendant la fenêtre temporelle considéré.

On détermine alors le signal synthétique en construisant une liste de motifs qui constituent le signal synthétique à partir des listes de motifs associées à chaque signal de mesure. Chaque motif de chacune des listes de motifs associées aux signaux de mesure est avantageusement pondéré par l'indice de confiance associé au signal de mesure associé pour l'instant temporel ou la fenêtre temporelle d'enregistrement associé(e) audit motif.

L'étape d'identification des motifs prédéfinis d'activité bioélectrique peut être mise en oeuvre à partir d'un ou plusieurs indicateurs calculés sur les signaux. Les indicateurs peuvent être par exemple choisis parmi la puissance du signal de mesure, la puissance de la dérivée du signal de mesure, les différentes bandes de fréquences du signal de mesure, la valeur de l'impédance du signal de mesure, le taux de passages par zéro du signal de mesure, la détection de saturations d'un amplificateur de la chaîne d'acquisition du signal de mesure, les valeurs du signal de mesure. Les indicateurs peuvent être calculés sur des fenêtres temporelles de durée variable, selon l'indicateur visé.

L'opération d'identification des motifs prédéfinis d'activité bioélectrique peut comporter la détermination d'un ou plusieurs indicateurs indiqués ci-dessus puis leur comparaison avec un ou plusieurs seuil(s) prédéfinis, par exemple à partir de valeurs usuellement rencontrées en électro-encéphalographie.

L'opération d'identification des motifs prédéfinis d'activité bioélectrique peut également employer un prédicteur obtenu par une méthode d'apprentissage (par exemple une forêt aléatoire ou un réseau de neurones) et entraîné sur une base d'apprentissage construite à partir d'extraits de signaux de mesure labellisés avec des motifs. Cette base d'apprentissage peut inclure des valeurs d'indicateurs (par exemple ceux présentés précédemment) calculés sur les mêmes signaux labellisés.

En particulier, l'opération d'identification des motifs prédéfinis peut prendre en compte le fait que l'évènement associé à un motif a une probabilité élevée pour plusieurs voies d'acquisition à un même instant temporel ou pendant une même fenêtre temporelle d'enregistrement.

En outre, l'opération d'identification peut favoriser une voie d'acquisition spécifique pour identifier un motif, l'évènement associé au motif étant plus susceptible d'être identifié par cette voie d'acquisition par rapport aux autres voies d'acquisition.

Enfin, on détermine continument le signal synthétique à partir des signaux de mesure et des séries temporelles d'indices de confiance. Cette étape va utiliser l'indice de confiance précédemment calculée ainsi que les listes de motifs identifiés sur les signaux de mesure.

Les probabilités des événements associés aux motifs précédemment calculées sur chacun des signaux sont par exemple pondérées par la qualité de ces signaux déterminée à partir de l'indice de confiance associé à l'instant temporel correspondant.

Une moyenne ainsi pondérée des détections de motifs permet de renforcer la robustesse des détections de motifs en tenant compte des différents canaux de mesure. Ici également les signaux ayant un indice de confiance inférieur à un seuil donné peuvent être écartés de la moyenne et la détermination du signal synthétique peut prendre en compte uniquement sur les signaux présentant un indice de confiance supérieur à un seuil fixé.

Dans un mode de réalisation particulier, il est possible de moyenner les signaux de mesure issus de plusieurs électrodes de référence (par exemple issus des deux mastoïdes) pour obtenir une super référence remplaçant lesdites références pour l'acquisition des signaux.

Une pondération au cours du temps de la moyenne permettant de calculer la super référence peut également être réalisée en fonction d'un indice de confiance déterminé par une analyse des signaux issus des électrodes de références comme cela a été détaillé ci-dessus.

## Revendications

1. Procédé de détermination d'un signal synthétique robuste indicatif d'une activité bioélectrique d'un individu dans lequel :
- on acquière continument au moins deux signaux de mesure (M) respectivement représentatifs de deux signaux électriques physiologiques d'un individu, au moyen d'au moins deux électrodes (2) placées en contact, ou en proximité, avec un individu,
- on construit continument, à partir des signaux de mesure (M) et au moyen d'une unité de traitement (3), au moins deux séries temporelles d'indices de confiance (C) respectivement associées auxdits signaux de mesure,
- on détermine continument un signal synthétique (S) à partir desdits signaux de mesure et desdites séries temporelles d'indices de confiance au moyen de ladite unité de traitement (3),
**caractérisé en ce que**,
1) pour déterminer continument le signal synthétique (S), on analyse continument chacun desdits signaux de mesure (M) pour identifier des motifs prédéfinis d'activité bioélectrique (P) et on associe à chacun desdits signaux de mesure une liste de motifs identifiés en analysant ledit signal de mesure, chaque motif identifié étant associé à un instant temporel ou une fenêtre temporelle d'enregistrement dans le signal de mesure (M) associé, et
2) pour chaque instant temporel ou fenêtre temporelle d'enregistrement dans le signal de mesure (M) associé, et pour chaque motif de la liste de motifs identifiés, on détermine une probabilité qu'un évènement associé au motif ait lieu.

2. Procédé selon la revendication 1, dans lequel chaque signal de mesure (M) est obtenu à partir d'une mesure différentielle entre deux électrodes de mesure (2a), entre une électrode de mesure (2a) et une électrode de biais (2b), ou encore entre une électrode de biais (2b) et une masse interne d'une unité de traitement (3) des signaux de mesure.

3. Procédé selon l'une quelconque des revendications 1 à 2, dans lequel les séries temporelles d'indices de confiance (C) sont construites en fonction d'au moins l'un parmi la puissance du signal de mesure (M), la puissance de la dérivée du signal de mesure (M), des puissances de bandes de fréquences du signal de mesure (M), un taux de passages par zéro du signal de mesure (M), une détection de saturations du signal de mesure (M).

4. Procédé selon l'une quelconque des revendications 1 à 3, dans lequel pour déterminer le signal synthétique (S), on détermine une série temporelle de valeurs de signal synthétique (S) telle que à chaque instant de la série temporelle, la valeur de signal synthétique correspond à la valeur du signal de mesure (M) associé à l'indice de confiance (C) le plus élevé pour ledit instant.

5. Procédé selon l'une quelconque des revendications 1 à 4, dans lequel pour déterminer le signal synthétique (S), on détermine une série temporelle de valeurs de signal synthétique telle que à chaque instant de la série temporelle, la valeur de signal synthétique correspond à une moyenne pondérée des valeurs des signaux de mesure (M), chaque valeurs des signaux de mesure étant respectivement pondérée en fonction de l'indice de confiance (C) associé au signal de mesure (M) pour ledit instant.

6. Procédé selon la revendication 1, dans lequel le signal synthétique (S) comporte une liste de motifs prédéfinis (P) d'activité bioélectrique associés à des instants temporels ou des fenêtres temporelles d'acquisition.

7. Procédé selon la revendication 6, dans lequel pour déterminer le signal synthétique (S), on construit la liste de motifs (P) constituant le signal synthétique à partir des listes de motifs associées à chaque signal de mesure, chaque motif de chacune desdites listes de motifs étant pondéré par l'indice de confiance (C) associé au signal de mesure (M) associé pour l'instant temporel ou la fenêtre temporelle d'enregistrement associé(e) audit motif.

8. Procédé selon l'une quelconque des revendications 1 à 7, dans lequel, on détermine une référence virtuelle à partir d'une mesure de potentiel d'une électrode de mesure (2a) ou d'une électrode de biais (2b) et en fonction d'une mesure d'un courant échangé entre une électrode de biais (2b) et l'individu (I).

9. Dispositif (1) d'acquisition d'un signal synthétique robuste indicatif d'une activité bioélectrique d'un individu comprenant :
- au moins deux électrodes (2) placées en contact, ou en proximité, avec un individu (I) pour acquérir continument au moins deux signaux de mesure (M) respectivement représentatifs de deux signaux électriques physiologiques,
- une unité de traitement (3) comprenant
- des moyens de réception (4) des signaux de mesure des électrodes, et
- des moyens de traitement (5) pour
construire continument, à partir des signaux de mesure (M), au moins deux séries temporelles d'indices de confiance (C) respectivement associées auxdits signaux de mesure,
déterminer continument un signal synthétique (S) à partir desdits signaux de mesure et desdites séries temporelles d'indices de confiance,
charactérisé en ce que,
pour déterminer continument le signal synthétique (S), chacun desdits signaux de mesure (M) est analysé continûment pour identifier des motifs prédéfinis d'activité bioélectrique (P) et, à chacun desdits signaux de mesure est associé une liste de motifs identifiés en analysant ledit signal de mesure, chaque motif identifié étant associé à un instant temporel ou une fenêtre temporelle d'enregistrement dans le signal de mesure (M) associé, et
pour chaque instant temporel ou fenêtre temporelle d'enregistrement dans le signal de mesure (M) associé, et pour chaque motif de la liste de motifs identifiés, une probabilité qu'un évènement associé au motif ait lieu est déterminé.

## Patentansprüche

1. Verfahren zur Bestimmung eines robusten synthetischen Signals, das eine bioelektrische Aktivität eines Individuums angibt, aufweisend:
- kontinuierliches Erfassen von mindestens zwei Messsignalen (M), die jeweils zwei elektrische physiologische Signale eines Individuums repräsentieren, mit Hilfe von mindestens zwei Elektroden (2), die in Kontakt mit oder nahe an einem Individuum angeordnet sind,
- kontinuierliches Konstruieren von mindestens zwei Zeitserien von Vertrauensindizes (C), die jeweils mit den Messsignalen verknüpft sind, ausgehend von den Messsignalen (M) und mit Hilfe einer Verarbeitungseinheit (3),
- kontinuierliches Bestimmen eines synthetischen Signals (S) ausgehend von den Messsignalen und den Zeitserien von Vertrauensindizes mit Hilfe der Verarbeitungseinheit (3),
**dadurch gekennzeichnet, dass**
1) zum kontinuierlichen Bestimmen des synthetischen Signals (S) jedes der Messsignale (M) kontinuierlich analysiert wird, um vorab definierte Motive bioelektrischer Aktivität (P) zu identifizieren, und mit jedem der Messsignale eine Liste von beim Analysieren des Messsignals identifizierten Motiven verknüpft wird, wobei jedes identifizierte Motiv mit einem Aufzeichnungszeitpunkt oder einem Aufzeichnungszeitfenster in dem verknüpften Messsignal (M) verknüpft ist, und
2) für jeden Aufzeichnungszeitpunkt oder jedes Aufzeichnungszeitfenster in dem verknüpften Messsignal (M) und für jedes Motiv der Liste identifizierter Motive eine Wahrscheinlichkeit, dass ein mit dem Motiv verknüpftes Ereignis stattfand, bestimmt wird,

2. Verfahren nach Anspruch 1, in welchem jedes Messsignal (M) aus einer Differenzmessung zwischen zwei Messelektroden (2a), zwischen einer Messelektrode (2a) und einer Bias-Elektrode (2b) oder auch zwischen einer Bias-Elektrode (2b) und einer inneren Erdung einer Messsignal-Verarbeitungseinheit (3) gewonnen wird,

3. Verfahren nach einem der Ansprüche 1 bis 2, in welchem die Zeitserien von Vertrauensindizes (C) als Funktion mindestens eines der folgenden Parameter konstruiert werden: die Stärke des Messsignals (M), die Stärke des Differentials des Messsignals (M), Stärken von Frequenzbändern des Messsignals (M), eine Rate von Nulldurchgängen des Messsignals (M), eine Detektion von Sättigungen des Messsignals (M).

4. Verfahren nach einem der Ansprüche 1 bis 3, in welchem zum Bestimmen des synthetischen Signals (S) eine Zeitserie von Werten des synthetischen Signals (S) derart bestimmt wird, dass an jedem Zeitpunkt der Zeitserie der Wert des synthetischen Signals dem Wert desjenigen Messsignals (M) entspricht, das für diesen Zeitpunkt mit dem höchsten Vertrauensindex (C) verknüpft ist.

5. Verfahren nach einem der Ansprüche 1 bis 4, in welchem zum Bestimmen des synthetischen Signals (S) eine Zeitserie von Werten des synthetischen Signals derart bestimmt wird, dass an jedem Zeitpunkt der Zeitserie der Wert des synthetischen Signals einem gewichteten Mittelwert der Werte der Messsignale (M) entspricht, wobei jeder Wert der Messsignale jeweils als Funktion des Vertrauensindexes, der für diesen Zeitpunkt mit dem Messsignal (M) verknüpft ist, gewichtet ist.

6. Verfahren nach Anspruch 1, in welchem das synthetische Signal (S) eine Liste von vorab definierten Motiven bioelektrischer Aktivität (P), die mit Erfassungszeitpunkten oder Erfassungszeitfenstern verknüpft sind, aufweist.

7. Verfahren nach Anspruch 6, in welchem zum Bestimmen des synthetischen Signals (S) die Liste von Motiven (P), die das synthetische Signal bildet, ausgehend von den mit jedem Messsignal verknüpften Motivlisten konstruiert wird, wobei jedes Motiv jeder der Motivlisten mit dem Vertrauensindex gewichtet ist, der mit dem Messsignal (M) verknüpft ist, das für den verknüpften Aufzeichnungszeitpunkt oder das verknüpfte Aufzeichnungszeitfenster mit dem Motiv verknüpft ist.

8. Verfahren nach einem der Ansprüche 1 bis 7, in welchem eine virtuelle Referenz ausgehend von einem Potentialmesswert einer Messelektrode (2a) oder einer Bias-Elektrode (2b) und als Funktion eines Messwerts eines zwischen einer Bias-Elektrode (2b) und dem Individuum (I) ausgetauschten Stroms bestimmt wird,

9. Vorrichtung zum Erfassen eines robusten synthetischen Signals, das eine bioelektrische Aktivität eines Individuums angibt, aufweisend:
- mindestens zwei Elektroden (2), die in Kontakt mit oder nahe an einem Individuum (I) angeordnet sind, um mindestens zwei Messsignale (M), die jeweils zwei elektrische physiologische Signale repräsentieren, kontinuierlich zu erfassen,
- eine Verarbeitungseinheit (3) aufweisend:
* Einrichtungen zum Empfangen (4) der Messsignale der Elektroden und
* Verarbeitungseinrichtungen (5) zum
kontinuierlichen Konstruieren von mindestens zwei Zeitserien von Vertrauensindizes (C), die jeweils mit den Messsignalen verknüpft sind, ausgehend von den Messsignalen (M),
kontinuierlichen Bestimmen eines synthetischen Signals (S) ausgehend von den Messsignalen und den Zeitserien von Vertrauensindizes,
**dadurch gekennzeichnet, dass**
zum kontinuierlichen Bestimmen des synthetischen Signals (S) jedes der Messsignale (M) kontinuierlich analysiert wird, um vorab definierte Motive bioelektrischer Aktivität (P) zu identifizieren, und mit jedem der Messsignale eine Liste von beim Analysieren des Messsignals identifizierten Motiven verknüpft wird, wobei jedes identifizierte Motiv mit einem Aufzeichnungszeitpunkt oder einem Aufzeichnungszeitfenster in dem verknüpften Messsignal (M) verknüpft ist, und
für jeden Aufzeichnungszeitpunkt oder jedes Aufzeichnungszeitfenster in dem verknüpften Messsignal (M) und für jedes Motiv der Liste identifizierter Motive eine Wahrscheinlichkeit, dass ein mit dem Motiv verknüpftes Ereignis stattfand, bestimmt wird.

## Claims

1. A method for determining a robust synthetic signal indicative of a bioelectric activity of an individual wherein:
- at least two measurement signals (M), which are respectively representative of two physiological electrical signals of an individual, are continuously acquired, by means of at least two electrodes (2) placed in contact, or in proximity, with an individual,
- at least two time series of confidence indices (C), which are respectively associated with said measurement signals, are continuously built, from the measurement signals (M) and by means of a processing unit (3),
- a synthetic signal (S) is continuously determined, from said measurement signals and said time series of confidence indices, by means of said processing unit (3),
**characterised in that**,
1) in order to continuously determine the synthetic signal (S), each of said measurement signals (M) is continuously analysed to identify predefined patterns of bioelectric activity (P) and a list of patterns, which are identified by analysing said measurement signal, is associated with each of said measurement signals, each identified pattern being associated with a time instant or a time window of recording in the associated measurement signal (M), and
2) for each time instant or time window of recording in the associated measurement signal (M), and for each pattern of the list of identified patterns, a probability that an event, which is associated with the pattern, takes place is determined.

2. The method according to claim 1, wherein each measurement signal (M) is obtained from a differential measurement between two measurement electrodes (2a), between a measurement electrode (2a) and a bias electrode (2b), or even between a bias electrode (2b) and an internal ground of a processing unit (3) of the measurement signals (M).

3. The method according to any one of claims 1 to 2, wherein the time series of confidence indices (C) are built depending on at least one of the power of the measurement signal (M), the power of the derivative of the measurement signal (M), the powers of frequency bands of the measurement signal (M), a zero-crossing rate of the measurement signal (M), a detection of saturations of the measurement signal (M).

4. The method according to any one of claims 1 to 3, wherein in order to determine the synthetic signal (S), a time series of synthetic signal values (S) is determined such that, at each instant of the time series, the synthetic signal value corresponds to the value of the measurement signal (M) associated with the highest confidence index (C) for said instant.

5. The method according to any one of claims 1 to 4, wherein in order to determine the synthetic signal (S), a time series of synthetic signal values is determined such that, at each instant of the time series, the synthetic signal value corresponds to a weighted average of the values of the measurement signals (M), each value of the measurement signals being respectively weighted depending on the confidence index (C) associated with the measurement signal (M) for said instant.

6. The method according to claim 1, wherein the synthetic signal (S) includes a list of predefined patterns (P) of bioelectric activity which are associated with time instants or time windows of acquisition.

7. The method according to claim 6, wherein in order to determine the synthetic signal (S), the list of patterns (P) constituting the synthetic signal is built from the lists of patterns which are associated with each measurement signal, each pattern of each of said lists of patterns being weighted by the confidence index (C) associated with the associated measurement signal (M) for the time instant or the time window of recording associated with said pattern.

8. The method according to any one of claims 1 to 7, wherein a virtual reference is determined from a potential measurement of a measurement electrode (2a) or a bias electrode (2b) and depending on a measurement of a current exchanged between a bias electrode (2b) and the individual (I).

9. A device (1) for acquiring a robust synthetic signal indicative of a bioelectric activity of an individual comprising:
- at least two electrodes (2) placed in contact, or in proximity, with an individual (I) to continuously acquire at least two measurement signals (M) which are respectively representative of two physiological electrical signals,
- a processing unit (3) comprising
- means (4) for receiving the measurement signals from the electrodes, and
- processing means (5) for continuously building, from the measurement signals (M), at least two time series of confidence indices (C) respectively associated with said measurement signals,
continuously determining a synthetic signal (S) from said measurement signals and said time series of confidence indices,
**characterised in that**,
in order to continuously determine the synthetic signal (S), each of said measurement signals (M) is continuously analysed to identify predefined patterns of bioelectric activity (P) and, a list of patterns, which are identified by analysing said measurement signal, is associated with each of said measurement signals, each identified pattern being associated with a time instant or a time window of recording in the associated measurement signal (M), and
for each time instant or time window of recording in the associated measurement signal (M), and for each pattern in the list of identified patterns, a probability that an event, which is associated with the pattern, will take place is determined.
